# EUROPEAN PATENT APPLICATION

(11) **EP 4 327 839 A1**
(43) Date of publication of application: **28.02.2024**
(21) Application number: 22191456.7
(22) Date of filing: 22.08.2022
(51) Int. Cl.: A61L 31/02

(54) **METHOD FOR PRODUCING VASCULAR IMPLANTS WITH A MAGNETIZED SURFACE**

(71) Applicant: Cortronik GmbH, 18119 Rostock-Warnemünde (DE)
(72) Inventor: Bayer, Ullrich, 18209 Bad Doberan (DE); Sannan, Ahmed, 17159 Dargun (DE); Grabow, Niels, 18055 Rostock (DE); Senz, Volkmar, 18069 Rostock (DE); Lebahn, Kerstin, 18057 Rostock (DE); Schmitz, Klaus-Peter, 18119 Rostock (DE)
(74) Representative: Biotronik Corporate Services SE

(57) **Abstract**

The invention relates to a method for producing a vascular implant from a vascular implant body with a surface, that is at least partially comprised of the chromium alloy, and the vascular implants obtained by this method.

## Description

The invention relates to a method for producing a vascular implant from a vascular implant body with a surface, that is at least partially comprised of the chromium alloy, and the vascular implants obtained by this method.

Vascular implants have been applied in the treatment of various medical conditions since the late 1980ies. The basic purpose of a vascular implant such as a graft or stent is to act as an artificial conduit or substitute for an anatomic vessel or hollow organ. Typical applications of such implants include the stenting of coronary arteries, of peripheral vessels such as the femoral arteries or of the colon as a palliative treatment.

Depending on the specific requirements of the respective application different materials such as plastics or metals are used. With regard to the metal-based vascular implants different groups of alloys exist, which can be classified into cobalt and iron-based alloys. The properties of the respective alloy are fundamentally determined by different elements present in the alloy, including chromium, platinum, nickel, molybdenum, tungsten and manganese as well as carbon and silicon and their respective ratios.

For example, molybdenum and tungsten are added to improve properties such as hardness, strength and toughness. The main function of chromium is to increase the corrosion resistance of the passive layer, which consists mainly of chromium oxides.

Current developments in the clinical and regulatory requirements aim for a further reduction of material usage and to minimize the release of clinically relevant metalloids such as cobalt ions. Alternatively, critical alloy constituents should be avoided altogether.

However, in light of the necessary physiological compatibility and mechanical requirements the desired miniaturization of the implant and/or substitution of individual components of the alloys is often of limited success. Furthermore, the manufacturing and processing methods for products such as stents made of these materials are considered to be highly developed and the potential for improvement deemed exhausted.

An alternative approach to achieve the goals outlined above are modifications to the implant surface, e.g., by the generation of one or more layers. Thereby, the existing very good volume properties of the established alloys can be retained, while simultaneously generating advantageous surface properties to fully guarantee the safety and functionality of the inserted vascular implant. In addition to increasing the wear and corrosion resistance, functional surfaces are of particular interest. For example, CA 2 637 662 A1 describes drug-eluting intravascular protheses. Said protheses are coated with a biodegradable drug-eluting polymer releasing an inhibitor of smooth muscle cell proliferation and a growth factor to prevent a restenosis and to enhance the tissue recovery following an intravascular intervention.

A major issue to overcome regarding drug-eluting implants is to assure a homogenous coating, which is not only required to obtain implants with a precise amount of the respective drug but also steadies the drug-release after its implantation. Furthermore, the coating must adhere sufficiently strong to the stent surface to avoid (partial) delamination of the layer(s). Whereas a coating may have a passivating effect at the beginning, said effect ceases upon degradation of said layer.

Another example for a functionalized surface are stents coated with magnetic nanoparticles as described by KR 102 062 700 B1. The application of an external magnetic field leads to an exothermic effect, i.e., heat emanates from said nanoparticles, which may be used for hyperthermal cancer therapy or other thermal based therapies. However, magnetization is only ensured as long as the coating is intact. Beyond that, the coating does not affect the mechanical properties of the base-alloy itself.

A more permanent solution are the stents comprising a ferromagnetic alloy with a relative magnetic permeability of more than 100 and a Curie temperature between 43 °C and 70 °C as taught by DE 101 13 659 A1. However, the ferromagnetic alloy of the stents may hamper imaging techniques such as MRI. As such, alloys for vascular implants are generally selected from non-magnetic alloys.

Therefore, it is the object of the present invention to provide a method for producing a vascular implant with a magnetized layer enhancing wear and corrosion resistance of the base alloy.

This object is solved by a method with the features of claim 1 comprising the steps
a) providing the vascular implant body comprising a chromium alloy having a surface, which is at least partially comprised of said chromium alloy,
b) subjecting the vascular implant body of step a) to at least one nitriding treatment and
c) magnetizing the vascular implant body obtained from step b) by means of an external magnetic field.

It was surprisingly found, that the vascular implants obtained by the above method feature a ferromagnetic layer comprising chromium(II) nitride (CrN). Said layer is formed in the nitriding step b) and allows the subsequent magnetization of the stent. Since the magnetization is only limited to the formed layer, imaging techniques commonly used in diagnostics are not impeded. Furthermore, the chromium nitride layer has a passivating effect and increases the hardness and wear resistance of the stent without affecting the properties of the base alloy.

The implant base body is obtained by standard methods known the skilled person. For example, one or more alloys, whereby at least one alloy comprises chromium, are (co-)extruded into tubes of the desired dimension. Thereby, surface of the tube and thus the later implant is at least partially comprised of the respective chromium alloy. The implant bodies are then produced from the tubes by laser cutting, deburring and pickling.

Preferably, the complete surface of the implant comprises chromium to ensure the formation of a homogenous surface in the later modification stages.

Whereas the method may be utilized for chromium alloys in general, only alloys, that are permissible for medical applications, should be used for the vascular implants. It is preferred that the alloy comprises at least 12 wt.-% chromium to ensure a sufficiently high concentration of chromium at and close to the surface, particularly preferably the chromium content is between 15 to 30 wt.-% of the alloy. The alloys 314LVM, L-605 and MP35N are especially suited due to their outstanding properties with regard to radiodensity, elasticity and the low strut thickness.

In a preferred embodiment the surface of the vascular implant is pickled and/or treated with an abrasive prior to step b). An example for a treatment with an abrasive is blasting the surface of the implant body with corundum. The abrasion and/or pickling results in an enlargement of the surface. The surface area can be determined by the standard N₂-BET adsorption method. Preferably the surface area is increased by a factor of least three, preferably by a factor between four to ten. The larger surface facilitates the nitridation process. Moreover, additional surface modifications of the stent such as coating are more feasible.

Suitable processes for the at least one nitriding treatment of step b) are plasma nitriding by glow discharge, nitrocarburation and/or gas nitriding. These processes are well known to a person skilled in the art. The plasma nitriding of alloys comprising chromium is described in CA 2 554 985 A1, for example. The nitriding requires the absence of oxygen to avoid the thermodynamically favored formation of chromium oxides. Nitriding using nitrocarburation is less preferred since chromium carbide do not exhibit the desired ferromagnetic properties.

In step b), chromium atoms present on at a part of the implant surface reacts with the nitrogen to CrN. Additionally, nitrogen diffuses into the alloy reacting with chromium near the surface. Thereby, a layer comprising CrN is formed. Further elements of the alloy such as iron or molybdenum may also react to the corresponding nitrides in dependency of the selected nitriding conditions. The formed layer increases of the surface hardness, prevents corrosion as well as constitutes a barrier against the diffusion of physiologically harmful ions from the alloy into surrounding tissue.

The advantage of the above-mentioned processes in comparison to other nitriding processes such as physical vapor deposition, reactive magneton sputtering or galvanic processes is that the CrN layer is formed from the base alloy instead of being deposited onto the implant surface. Considering the diffusion of nitrogen into the alloy, a thin transition layer runs in between the CrN layer and the unchanged alloy rather than a clear boundary. Consequently, flaking is effectively prevented. Moreover, the implant's properties are modified while keeping its dimensions essentially unchanged in contrast to deposition techniques that obviously increase the dimensions.

In addition to material savings, thinner implant struts also have further advantages such as a significant increase of the flexibility, which facilitates stent placement in the vessel, for example. Furthermore, these implants show a better ingrowth behavior, since a smaller amount of foreign material is introduced into the human body and is in contact with the vessel wall.

Important mechanical properties of the vascular implants such as the elongation at break, the tensile strength, the modulus of elasticity or the radial force may be determined by standardized test methods according to DIN EN ISO 25539-2 (01.2021) involving static or dynamic tensile tests as well as fatigue tests, for example.

In a preferred embodiment the nitriding treatment of step b) is a gas nitriding process conducted at a temperature of 450 to 1100 °C and a nitrogen pressure between 0.25 to 2.0 bar of nitrogen. Preferably, the temperature is selected from a range between 500 and 850 °C, particularly preferably from between 520 to 700 °C. In a preferred embodiment the nitrogen pressure is between 0.5 and 1.0 bar.

These conditions allow the controlled nitriding of the implant surface in dependency of the thermal properties of the respective alloy such as its melting point. Thereby, higher N₂ pressures and higher temperatures generally lead to a faster nitriding reaction as well as an enhanced diffusion of nitrogen deeper into the implant body. Furthermore, higher temperatures, in particular above 1050 °C, lead to a progressing conversion of CrN to chromium(I) nitride, Cr₂N. In comparison to CrN, Cr₂N leads to a further hardening of the surface albeit at the expense of the oxidation resistance, allowing to adapt the protective layer's properties. Nevertheless, only CrN exhibits a magnetizable phase.

Considering the small dimensions of the implant, the duration of step b) is less than 4 hours, preferably 30 min to 2 hours, in dependency of the selected temperature, nitrogen pressure and the targeted layer thickness (*vide infra*)*.* The surface thickness as well as its composition can be determined by scanning electron microscopy in combination with energy dispersive X-ray spectroscopy.

The magnetization of the implant surface is achieved by techniques known to persons skilled in the art, such as by the application of external electric and/or magnetic fields. Whereas the bulk chromium nitride exhibits a magnetic transition from antiferromagnetic to paramagnetic above 0 °C, layers of CrN are ferromagnetic, which allows a magnetization of the CrN layer of the vascular implant using an external magnetic field. Since the base alloy is not magnetic, only the thin surface layer is affected by external magnetic fields.

An important measure describing how much a material will become magnetized in an applied magnetic field is the magnetic susceptibility *X*. It is the ratio of magnetization M (magnetic moment per unit volume) to the applied magnetizing field intensity H. Paramagnetic materials are characterized by a *X* > 0 in an order of magnitude of approximately 10⁻³. Diamagnetic substances exhibit a susceptibility *X* < 0. In contrast, magnetic susceptibility of ferromagnetic materials is >> 1. The behavior of ferromagnetic materials is often described by a hysteresis curve that may be determined by means well known to the skilled person such as magnetometer. The susceptibility may be measured with an Evans balance.

The level of magnetic susceptibility is an easily identifiable distinguishing feature (possibly also to competing products) and represents a possibility for product data storage via UDI (Unique Device Identification). Furthermore, implants featuring magnetism exhibit an improved ingrowth behavior and correspondingly lower risk of thrombosis. For this purpose, endothelial progenitor cells (EPC) are to be bound to the stent in order to restore normal blood flow through the corresponding blood vessel as quickly as possible. This process is promoted by magnetism.

In a preferred embodiment, the external magnetic field is generated by means of a permanent magnet, preferably a neodymium magnet. Permanent magnets are characterized by a constant magnetic field, which allows to reproducibly magnetize the vascular implants obtained from step b). Thereby, neodymium magnets are the strongest type of commercially available permanent magnets, facilitating the magnetization process.

The stents are magnetized with a neodymium magnet. Measurements have shown that the magnetized stents maintain a magnetic field in the range of 50 to 500 mG. In comparison, a non-magnetized stent is in the range of 10 mG.

In a further embodiment, the vascular implant of step c) is subjected to a coating treatment with a coating solution comprising polymeric capsules, whereby the polymeric capsules comprise ferromagnetic particles and at least one drug. Thereby, capsules comprising the ferromagnetic particles will adhere to the implants surface due to its magnetization allowing the elution of the respective drug.

The coating treatment comprises one or more dipping and/or spraying steps to ensure that a sufficient amount of the at least one drug is loaded onto the implant. The at least one drugs is previously enclosed in a polymeric coating material containing nanoscale ferromagnetic particles. The polymer is preferably selected from bioresorbable polymers, particularly from polylactic acid, polycarbonate or salicylic acid polymers.

The drug is mainly to inhibit neointimal growth (due to the proliferation of smooth muscle cells) that could cause restenosis. Hence, immunosuppressive and/or antiproliferative drugs such as sirolimus, paclitaxel or everolimus are used.

The ferromagnetic particles are preferably selected from biocompatible components such as Fe₃O₄ or Gd. In contrast to standard polymeric coating, the undesired effect of overspray is significantly reduced, since the adhesion is governed by attractive magnetic forces. Thus, the amount of polymeric carrier material can be significantly reduced.

In a preferred embodiment, the coated vascular implant is subsequently rinsed and a subjected to a final annealing process improve adhesion.

Optionally, the implants treated in this way can then be subjected to a short spray process with pure polylactic acid for better drug fixation.

The invention further relates to vascular implants comprising a chromium alloy obtained by the method described above. Thereby, at least part of its surface is a layer comprising magnetized, ferromagnetic chromium nitride. The CrN of the layer enhances the surface hardness as well as the wear and corrosion resistance. These improved mechanical characteristics of the implant allow a further miniaturization, i.e., by achieving the same or better characteristics as untreated vascular implants based on the same alloy as with less material. Furthermore, the magnetization improves ingrowth properties as outlined above and allows thermal treatments without impeding imaging techniques such as MRI.

In a preferred embodiment, the complete surface is comprised of the layer comprising magnetized, ferromagnetic chromium nitride. Thus, the beneficial effects of the CrN layer applies to the complete implant.

Preferably, the chromium alloy is an alloy with a chromium content of at least 12 wt.-% and comprises Co, Fe, Mo, Mn, Ni and/or mixtures thereof. A minimum chromium content of 12 wt.-% ensures a sufficiently high concentration of chromium at the surface and in surface-near region. The further elements are commonly used for vascular implants and allow to specifically adapt the mechanical and physiological properties of the alloy. Thereby, a chromium content of 15 to 30 wt.-% is particularly preferred. The alloys 314LVM, L-605 and MP35N are especially suited due to their outstanding properties with regard to radiodensity, elasticity and the low strut thickness.

In a further embodiment, the layer of magnetized, ferromagnetic chromium nitride has a thickness of 2 to 20 µm, preferably 3 to 10 µm. The thickness of the layer should be below the given threshold, since the corrosion and wear resistance will hardly be improved. On the other hand, a (too) thick CrN comprising layer may adversely affect the properties of the vascular implant, particularly if the characteristics of the layer predominates in comparison to the base alloy. As such the thickness of the CrN comprising layer has to be adjusted to the respective vascular implant, whereby typical values for the thickness of a strut of an implant range between 50 and 250 µm. The ratio of the strut thickness to the layer thickness should be between 25:1 and 5:1. The ratio can be determined by electron scanning microscopy, whereby the comparatively thin transition layer between the bulk alloy and the CrN-layer is neglected.

In a preferred embodiment, the vascular implant comprises one or more layers comprising polymeric capsules, wherein said layers adhere to the layer comprising magnetized, ferromagnetic chromium nitride and the polymeric capsules comprise ferromagnetic particles and at least one drug. Such vascular implants qualify as drug-eluting implants.

Thereby, polymer of the capsules is preferably selected from bioresorbable polymers, particularly from polylactic acid, polycarbonate or salicylic acid polymers. The capsules will slowly be resorbed *in vivo* thereby locally releasing the drug to promote healing of the tissue and prevent restenosis.

The at least one drug is preferably selected from immunosuppressive and/or antiproliferative drugs including but not limited to drugs such as sirolimus, paclitaxel or everolimus. The ferromagnetic particles are preferably selected from biocompatible components such as Fe₃O₄ or Gd and cause the adherence of the capsules to the magnetized implant surface.

Preferably, the surface of the implant is structured, i.e., features an enlarged surface, such that a larger number of polymeric capsules and thus, larger amount of drugs adhere to the implant.

Further objectives, features, advantages and possible applications of the invention can also be taken from the description of the following example. All features described form the subject matter of the invention per se or in any combination, independent of their inclusion in the claims or their back references.

### Example

Commercially available cobalt-chromium alloys (L 605, CoCr29Mo6) are each pressed into a tube using known extrusion methods. The obtained tubes have the dimensions 1.80 x 0.090 mm, 2.00 x 0.150 mm, 3.00 mm x 0.140 mm or 6.00 mm x 0.250 mm (outside diameter x wall thickness). The vascular implant bodies are produced from the tubes by laser cutting, deburring and pickling, which are then blasted using abrasively acting particles. The semi-finished products produced in this way, with increased surface area compared to polished semi-finished products, are heated to 650 °C at 0.5 bar nitrogen for to 2 hours leading to a surface comprising CrN with a thickness of 6 to 8 µm. After cooling, the surface-modified implants are magnetized using a neodymium magnet.

## Claims

1. Method for producing a vascular implant comprising the steps
a) providing the vascular implant body comprising a chromium alloy having a surface, which is at least partially comprised of said chromium alloy;
b) subjecting the vascular implant body of step a) to at least one nitriding treatment and
c) magnetizing the vascular implant body obtained from step b) by means of an external magnetic field.

2. Method according to claim 1, wherein the surface of the vascular implant is pickled and/or treated with an abrasive prior to step b).

3. Method according to claim 1 or 2, wherein the nitriding treatment of step b) is a gas nitriding process conducted at a temperature of 500 to 1100 °C and a nitrogen pressure between 0.25 to 2.0 bar of nitrogen.

4. Method according to any of the previous claims, wherein the external magnetic field is generated by means of a permanent magnet, preferably a neodymium magnet.

5. Method according to any of the previous claims, wherein the vascular implant of step c) is subjected to a coating treatment with a coating solution comprising polymeric capsules, whereby the polymeric capsules comprise ferromagnetic particles and/or at least one drug.

6. A vascular implant comprising a chromium alloy obtained by method according to any of claims 1 to 5, **characterized in that** at least part of its surface is a layer comprising magnetized, ferromagnetic chromium nitride.

7. A vascular implant according to claim 6, wherein the chromium alloy is an alloy with a chromium content of at least 12 wt.-% and comprises Co, Fe, Mo, Mn, Ni and/or mixtures thereof.

8. A vascular implant according to claims 6 or 7, **characterized in that** the layer of magnetized, ferromagnetic chromium nitride has a thickness of 2 to 20 µm, preferably 3 to 10 µm.

9. A vascular implant according to any claims 6 to 8, **characterized in that** the vascular implant comprises one or more layers comprising polymeric capsules, wherein said layers adhere to the layer comprising magnetized, ferromagnetic chromium nitride and the polymeric capsules comprise ferromagnetic particles and at least one drug.
